# EUROPEAN PATENT APPLICATION

(11) **EP 1 714 668 A1**
(43) Date of publication of application: **25.10.2006**
(21) Application number: 06008447.2
(22) Date of filing: 24.04.2006
(51) Int. Cl.: A61M 16/16, A61M 16/00, G06F 19/00, A61M 16/10

(54) **Breathing assistance apparatus**

(30) Priority: 22.04.2005 NZ 53964005
(71) Applicant: Fisher & Paykel Healthcare Limited, East Tamaki, Auckland (NZ)
(72) Inventor: Makinson, Ian Douglas, Auckland (NZ); Wixey, David, Auckland (NZ)
(74) Representative: Hoarton, Lloyd Douglas Charles

(57) **Abstract**

A breathing assistance apparatus that comprises a gas delivery device adapted to provide gas at a substantially positive mean pressure. The gas delivery device includes a communication port adapted to communicate with an external device and the device is configurable to at least two configurations. The first configuration is where the gas delivery device has a predetermined functionality in a first state. The second configuration is where the gas delivery device has a predetermined functionality in a second state. The second configuration is activated by the external device. The external device may be a mechanical key or a software key.

## Description

### FIELD OF INVENTION

The present invention relates to humidification particularly though not solely to humidifying gases to a user requiring Continuous Positive Airway Pressure (CPAP).

### SUMMARY OF THE PRIOR ART

It is known in the art to provide CPAP treatment in conjunction with humidity, for example, United States Patent Number 6,050,260. The humidification is usually provided either by:
1. An integrated CPAP blower and humidifier as described in US 6,050,260, whereby there is no separation of the CPAP and heated humidifier except for when the humidification chamber is removed for filling and cleaning.
2. A stand alone CPAP device connected by a flexible airway tube to a standalone heated humidifier, the equipment generally mounted on a tray for stability.
3. A CPAP device that can stand alone but can also be attached to a modular heated humidifier. On such a device, neither a flexible airway tube nor a mounting tray is required.

It would be desirable to provide a CPAP device which could be easily upgraded to provide humidification of gases supplied by the CPAP device.

An example of an upgradeable gas device is United States Patent Application No. 2002/0077856 assigned to Respironics, Inc.

### SUMMARY OF THE INVENTION

It is an object of the present invention to provide a breathing assistance apparatus that may be upgraded or at least provides the healthcare industry with a useful choice.

Accordingly in a first aspect the invention consists in a breathing assistance apparatus comprising:
a gas delivery device adapted to provide gas at a substantially positive mean pressure;
a communication port adapted to communicate with an external device;
   wherein said device is configurable to at least two configurations,
   a first configuration where said gas delivery device has a predetermined functionality in a first state, and
   a second configuration where said gas delivery device has a predetermined functionality in a second state, said second configuration being activated by said external device.

Preferably said external device is rendered inoperable if said second configuration is activated.

Preferably said gas delivery device includes a humidifier for humidifying said gas.

Preferably said predetermined functionality is active humidification of said gas.

Preferably a portion of said apparatus is excluded from use in said first configuration, whereby the integration of use of said portion to said apparatus corresponds to said second configuration.

Preferably said second configuration is enabled by connecting a software or hardware upgrade key to said apparatus.

Preferably said upgrade key includes an electronic circuit or controller configured to provide a predetermined response data stream to a received predetermined request data stream.

Preferably said electronic circuit or controller includes at least one register indicating whether said electronic circuit or controller has been used and if it so said circuit or controller is rendered inoperative for subsequently activating said second configuration.

Preferably said register includes the serial number of the apparatus said electronic circuit or controller was used with.

Preferably said apparatus further comprises a conduit between said gas delivery device and a patient and said predetermined functionality is active humidification and the provision of a heater within or incorporated with said conduit.

Preferably said predetermined functionality relates to the heating said gas and said second configuration relates to energising said heater to heat said conduit and/or said gas directly.

Preferably said predetermined functionality relates to storage or display of data relating to the use of said apparatus and said second configuration relates to energising a display to indicate said use.

Preferably said predetermined functionality relates to the pressure level of said gases delivered to the patient and said second configuration relates to providing either a continuous or stepwise correction in said pressure level.

In a second aspect the present invention consists in a method of delivering respiratory gases to a patient comprising the steps of:
providing gases at a substantially positive mean pressure from a gases supply device;
said gases supply device communicating with an external device to enable the switching of said gases supply device between at least two configurations, a first configuration with a predetermined functionality in a first state, and
a second configuration with said predetermined functionality in a second state capable of being enabled by said external device.

Preferably said method includes the step of said external device being rendering inoperative if said second configuration is enabled.

The invention consists in the foregoing and also envisages constructions of which the following gives examples.

### BRIEF DESCRIPTION OF THE DRAWINGS

Preferred forms of the present invention will now be described with reference to the accompanying drawings.
Figure 1 is a perspective view of a breathing assistance apparatus of the present invention in a CPAP only configuration.
Figure 2A is a blown out view of the CPAP only configuration of Figure 1.
Figure 2B is a reverse angle of Figure 2A.
Figure 3 is a perspective view of the breathing assistance apparatus of the present invention in a first form of a humidified CPAP configuration with a first form of a control panel.
Figure 4 is a front view of a breathing assistance apparatus in the humidified CPAP configuration with a second form of a control panel.
Figure 5 is a front view of a breathing assistance apparatus in the humidified CPAP configuration with a third form of a control panel.
Figure 6 is a block diagram of the dongle hardware.
Figure 7 is a memory array for the dongle.
Figure 8 is a flow diagram for the dongle operation.
Figure 9 is a flow diagram for the CPAP device operation.
Figure 10 is an exploded view of an example of an electronic upgrade key or dongle that may plug into a serial port of a humidifier.
Figure 11 is a perspective view of the key or dongle of Figure 10 plugged into a humidifier.

### DETAILED DESCRIPTION

A breathing assistance apparatus is described below that comprises a gas delivery device adapted to provide gas at a substantially positive mean pressure. The gas delivery device includes a communication port adapted to communicate with an external device and the device is configurable to at least two configurations. The first configuration is where the gas delivery device has a predetermined functionality in a first state. The second configuration is where the gas delivery device has a predetermined functionality in a second state. The second configuration is activated by the external device. The external device may be a mechanical key or a software key.

The present invention may include an integral, separable or separate humidifier which may be selectively configured to an inoperative or operative state. The switch over between use with a humidifier and without is dependent on the user or the seller having access to an actuation tool (described later) to activate the humidifier.

A typical integrated CPAP humidifier is described in United States Patent No. 6,050,260. The contents of which are incorporated herein by reference.

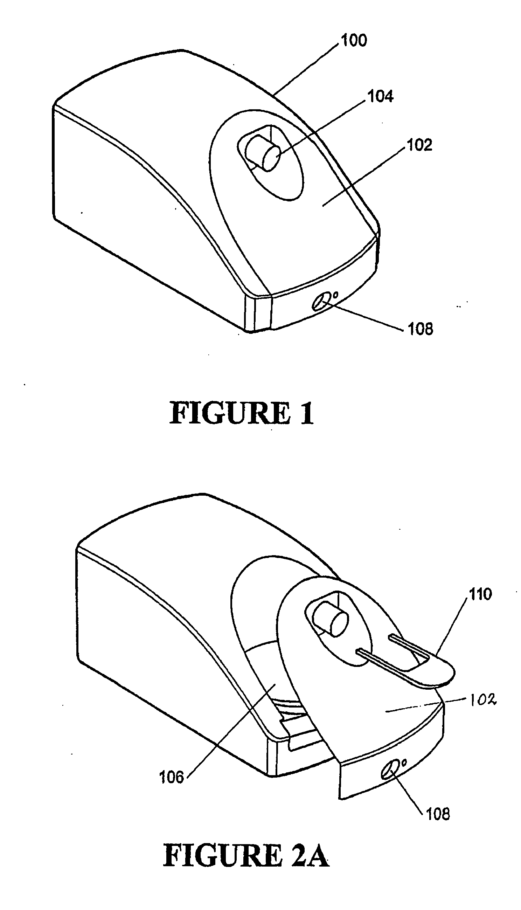

Referring to Figure 1 the CPAP device 100 is initially available for use as a standalone CPAP. The heater plate is isolated by a covering shroud 102; the shroud also forms a connection port for the CPAP device outlet 104. In this configuration the heated humidification hardware and control are disabled.

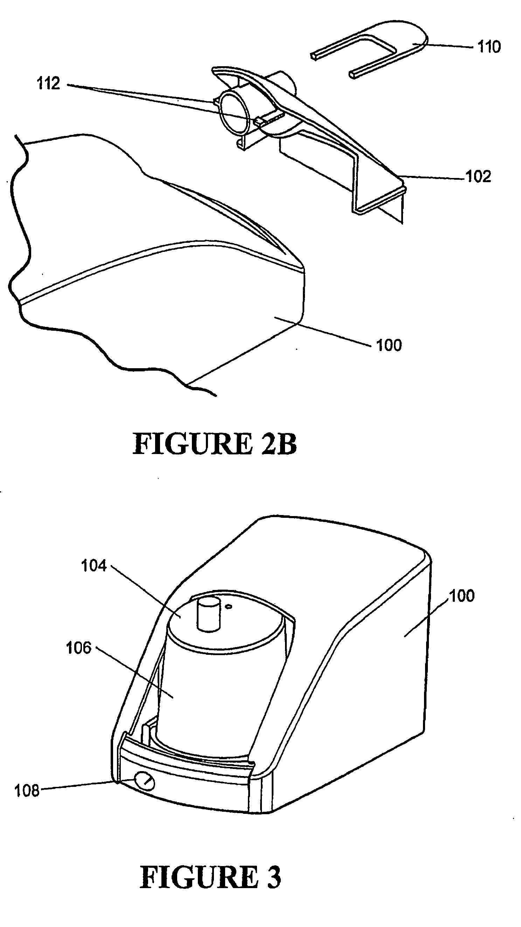

Upgrade to a CPAP device and activation of the control of hardware including heated humidification is completed by the installation of an actuation tool. Removal of the heater plate isolating the shroud 102 shown in Figure 3, and/or installing the actuation tool enables the software controlled heated humidification hardware 106 for heated humidifier operation.

Examples of the various forms of actuation tool may include:

### 1. Software key via serial data port

The CPAP device may be connected via an RS232 serial connection to a computer or directly via a TCP/IP or telephone line to the Internet to receive either additional software or coded actuation data.

### 2. Mechanical Key

The heater plate vestibule is covered by a shroud 102. A mechanical key (110, Figure 2A) is used to remove the shroud. The heater plate control knob 108 is locked off by the shroud 102 as shown in Figure 2A. The upgrade kit may include a knob to turn the heater plate on. In one embodiment, shown in Figures 2A & 2B, engaging the key 110 pushes two snap fit flanges 112, allowing the shroud 102 to be removed.

### 3. Magnetic Key

Similar to the mechanical key a magnetic key may be used to remove a shroud, as in Figure 2. The key preferably has magnets in a predetermined pattern. This key is slid into a cavity in the case of the CPAP device. Inside the case there are Hall effect sensors that detect the pattern of magnets. If the key is detected, the heater plate is activated.

### 4. Code Number Verification

a) This verification can be illustrated using Figure 2. A code number may be entered into the CPAP device. Particularly, a dealer of the CPAP device or user may phone a free phone number with the serial number of the CPAP device and pays for the upgrade. The freephone service then gives the dealer or user a code number that is specific to the serial number of the CPAP device. This code number may then be entered into a keypad (not shown) to activate the humidifier part of the CPAP device. The free phone service could alternatively be an Internet based service.
b) Another alternative is that the upgrade kit may include a card with a number hidden inside the packaging of the CPAP device. This number may give a free phone service evidence that the dealer has paid for an upgrade kit, and a financial transaction then doesn't have to be conducted on the phone or internet. Similarly to prevent fraud the numbers could be stored such that if a number is used twice an alarm is raised. A database of humidifier serial numbers and codes that have been sold may also be required. Also the numbers that have been used for an upgrade may also be stored in order to prevent a dealer or user making up numbers.
c) Another alternative would be for the dealer or user to provide the humidifier serial number and upgrade pack serial number. If both are valid then a pin may be generated by an algorithm using the humidifier serial number. A central database would keep a record of which upgrades had been used in relation to each humidifier. The serial number of the upgrade kit may follow some preset pattern for example only numbers that are divisible by, for example 13 could be used. This means that maintaining a database of serial numbers sold may not be required. The chance of the dealer or user making up a valid number in the example is 1 in 13 which should be sufficient to deter him or her, especially if recording any invalid attempts was included.

The algorithm may alternatively convert the serial number into a 4 digit number which is the PIN number.

### 5. Dongle

A plug in pack or "dongle" containing an electronic circuit designed to respond in a predetermined way to interrogation by the CPAP device (slave device), or a plug in pack containing an electronic circuit designed to interrogate and control the CPAP device (master device) may be supplied with the CPAP device. The plug in pack may plug into a dedicated socket in the device, or into the existing serial port connection point. Similarly, a smart card of know type could be interfaced with the CPAP device to activate the humidifier.

Additionally, such a dongle may contain some of the electronic circuit needed to operate the heater plate of the humidifier part of the CPAP device. Again a shroud as seen in Figures 1 and 2 would be required.

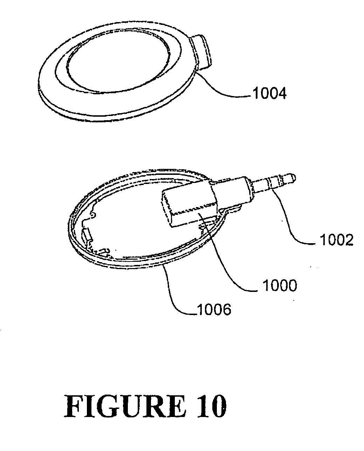

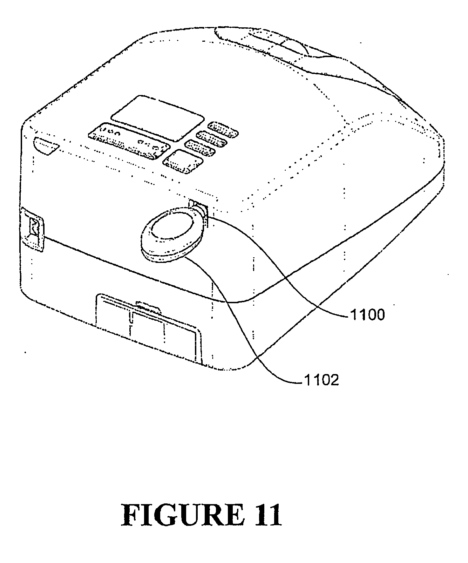

A further example of an electronic upgrade key or dongle that plugs into a serial port on the humidifier part of the CPAP device as seen in Figure 10. The key or dongle is shown in Figure 10 and includes a plug 1002, electronic circuit 1000, lower housing 1006 and upper housing 1004. The overall unit 1102 is then plugged into a socket 1100 as shown in Figure 11.

### 5.1 Function

In use, to upgrade the CPAP device to allow humidification and heating of gases, a master device upgrade key or dongle (a master device) is preferably plugged into the CPAP device while the CPAP device is switched on and operating. Upon connection, the upgrade key or dongle issues a unique serial command to the CPAP device to enable humidifier functionality. Once this command has been processed by the CPAP device, the humidifier is permanently enabled on the CPAP device. Consequently the presence of the upgrade key or dongle is no longer required. The advantage of this design is that the upgrade key or dongle may be used in an existing serial port of the CPAP device without depriving the port of other uses.

Alternately, a slave device upgrade key or dongle may first be plugged into the CPAP device before the CPAP device is switched on. The CPAP device may be designed to only check for the presence of a slave upgrade key once during power-up. The advantage of this design is that such an upgrade key may be used in the existing serial port of the CPAP device without depriving the port of other uses. It is of course conceivable that the CPAP device may be provided with a dedicated serial port for the slave upgrade key and, as such, the CPAP device can be adapted to always check that port for the presence of an upgrade key.

Additionally, once the CPAP device is switched on with the upgrade key plugged in, the user may hold down a combination of buttons to indicate the user's desire to upgrade. Once the upgrade key is detected and verified by the CPAP device, the CPAP device or upgrade key initiates the required configuration change.

In the preferred form the upgrade key or dongle preferably has two modes of operation. The first mode of operation is activated when the key is connected to a CPAP device ("normal mode"). The second mode of operation is activated when the key is connected to a computing device ("factory mode"). In the normal mode, the key acts as a master, where it issues commands to the CPAP slave device and monitors the data returned from the CPAP slave device. In the factory mode, the key acts a slave, where it services requests generated by certain software on the computing device. This allows the configuration of the key to be queried and modified by the computing device during manufacturing and/or servicing.

As noted above, the upgrade key could be used to upgrade the CPAP device by activating the humidifier in the device. Alternatively, the upgrade key could be used to customise and configure parameters of the CPAP device, also known as 'remote configuration'. For example, the CPAP manufacturer may produce the device with a standard default setting to the device. To tailor the CPAP device to a certain customer's requirement, the manufacturer may produce an upgrade key programmed with the customer's requirements. Example parameters that may be programmed include the gases pressure setting, altitude setting, humidity comfort level and gases deliver tube setting.

Another preferred feature of the upgrade key is the ability to refurbish a rental or second-hand CPAP device by restoring the original factory settings in a process known as 'factory reset'. This could be done by connecting the upgrade key to the CPAP device and allowing the key to reprogram or reset the CPAP device. As the key only performs a reset to factory settings, the key may be reused without being deactivated. Such a key would be targeted at dealers and distributors servicing CPAP devices.

### 5.2 Construction

In one form, the upgrade key may have a loop-back inverter design. This design employs a simple inverting circuit that is placed between transmit and receive lines when the upgrade key is connected to CPAP device. When the CPAP device transmits a code sequence, an inverted echo is generated from the key and sent back to the CPAP device. This inverted echo is verified by the CPAP device and the CPAP configuration is altered depending on the result of the verification.

Due to this simple design, the cost of a loop-back inverter upgrade key is relatively low. The simple nature of the design is, however, susceptible to circumvention by a user. Furthermore, the key cannot be deactivated once it has been read. This means that either the key must be permanently attached to the CPAP device, or potentially available to unlock multiple CPAP devices, which is undesirable for certain upgrade features.

In a preferred form, the upgrade key incorporates a microcontroller. When the upgrade key is connected to the CPAP device, a particular serial command signal is transmitted to the CPAP device to initiate the desired upgrade operation. The command signal is received by the CPAP device and verified. If the correct signal is received, the CPAP device carries out the desired configuration change internally, for example to allow upgrade features to be added to the CPAP device.

For the microcontroller-form upgrade key, the requirement for a microcontroller and its associated circuitry leads to a relatively high cost of manufacture. However, this is balanced by an improved security level that prevents circumvention by a user. The microcontroller key can also be deactivated to prevent the same key from being used to configure other CPAP devices. For instance, once the desired upgrade operation has been verified to have been completed, the microcontroller may set a special non-volatile memory flag to indicate that no further upgrades should be performed, this will be described later.

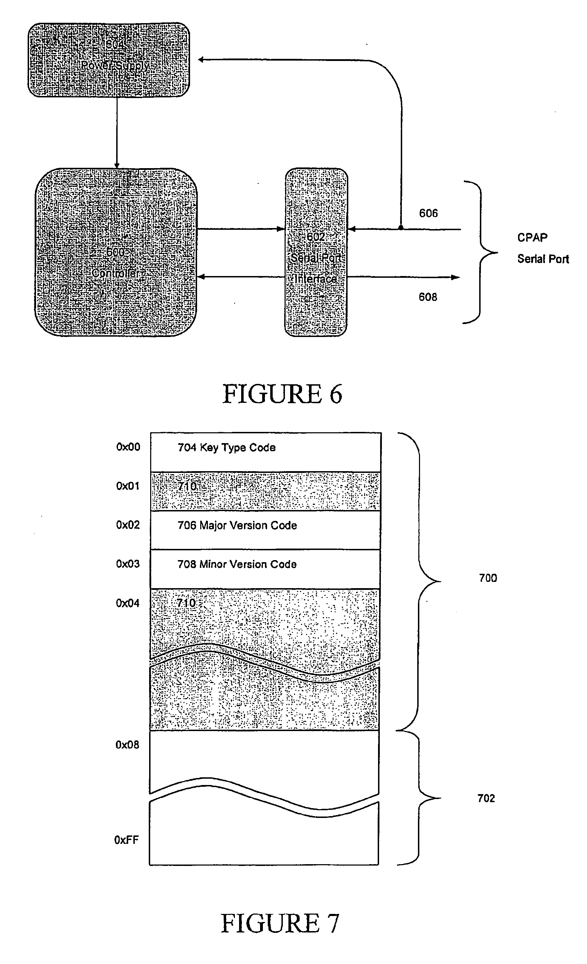

Figure 6 shows an example connectivity of the microcontroller-form key. In the most preferred form, the microcontroller 600 is a PIC12F629, which is an 8-bit, 4MHz processor. The microcontroller 600 is connected to a simple serial port interface 602, which implements the external communications port for the key. The serial port interface specification is a common one, defined as 9600 baud (or bits per second); with one start bit, one stop bit, eight data bits and no parity. Power to operate the microcontroller 600 comes from a parasitic power supply 604. The power supply is called this because it extracts the power required from the same serial port interface that is used for communications. In particular, power is extracted from the transmit line 606. The transmit line 606 and receive line 608 are connected to a CPAP serial port shown generally as 610.

In preferred form the upgrade key may also include an internal 256 byte non-volatile memory array, which is used to store configuration and status information about the key. An example structure of the non-volatile memory array is shown in Figure 7. Details of the memory array will be described below. The details are only exemplary and are not the only ways in which the memory array may be provided.

The array is preferably divided into two distinct regions: a control block 700 and a user block 702. The control block 700 constitutes the first eight bytes of the memory space and is a fixed read-only section. Each of the bytes in this block are allocated to a specific use as will be described. The key-type code 704 contains a single-byte value that identifies the type of key, or what the key is programmed to do. Examples of key types have been described above as the humidifier upgrade, remote configuration and factory reset.

The next field in the control block 700 occupies two locations to store the version information for the firmware contained in the key. The first byte in the field, major version code 706, stores the major key version number as a binary integer. This is incremented each time a major change to the software is made. The second byte, minor version code 708, contains the minor version number, also as a binary integer, and this is incremented whenever only small changes are made.

The remaining locations in the control block memory space are marked as reserved, shown as 710, and cannot be used by either the upgrade key firmware or a key reader device, such as a personal computer system.

The user block 702 is a general area that can be used to store status and configuration information of each key type. Consequently, the specific allocation of bytes in this space depends on the type of key that is installed. For example, for a humidity upgrade type key, the first eight locations in the block 702 are used to store the serial number of the CPAP device that was last attempted to be upgraded. This is followed by a single-byte location that stores the single-use status of the function. If this location contains the value zero, the key has been deactivated and will no longer perform upgrades.

### 5.3 Operation

### 5.3.1 Command Structure

To understand how the upgrade key of the preferred operates, an example command sent from the upgrade key to the CPAP device to enable the humidifier function, will firstly be described. The system uses a simple ASCII-based command structure to do this. All the commands are initiated by the upgrade key master, and processed by the CPAP device slave. An example command is shown below:
**E 0 1 3 \ r \ n**

A command might consist of a four character string followed by a set of '\r\n' terminating characters. This string can be separated into two parts: the first of which contains a single character unique identifier code and the second a three character numerical parameter. These two pieces of information are used by the CPAP software to process the command and initiate the desired operation.

The unique command identifier, shown as 'E', specifies the operation that is desired to be performed. The second parameter specifies any additional information required by the command. Here 'E' could be the command for performing a factory reset operation.

### 5.3.2 Upgrade Key Command Issuing

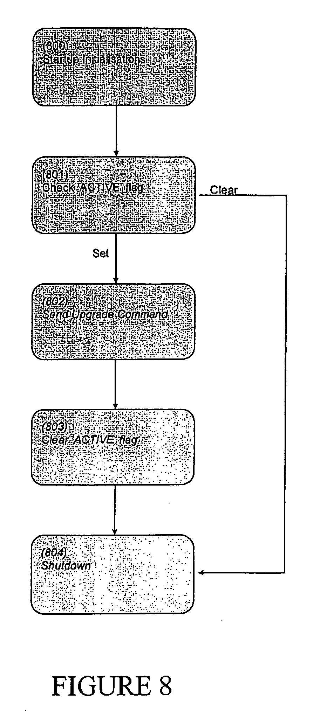

The operation of the upgrade key device when connected to a CPAP unit is shown in Figure 8. Upon connection, the upgrade key receives power from the CPAP device. This starts up the device's microcontroller system, allowing it to perform any required start-up initialisations (800). Following this, the upgrade key interrogates (801) its internal ACTIVE flag, which is stored in non-volatile memory. This is used to implement the single use functionality of the device.

If this flag is cleared, the upgrade key is said to be deactivated, and immediately enters shutdown mode (804). This ensures that the key is only capable of upgrading a single CPAP device. Alternately, if this flag is set, the upgrade key is said to be active and proceeds to step 802. This involves issuing a serial command to the CPAP device. The CPAP then recognises this command and enables the humidity controls internally.

Once the command has been sent, the upgrade key device proceeds to clear the 'ACTIVE' flag (803). The device is now deactivated, and cannot be used to upgrade further CPAP devices.

At the conclusion of these events, the upgrade key device enters a shutdown mode (804). In this mode the systems microcontroller is no longer active and no further operations are performed.

### 5.3.3 CPAP Command Processing

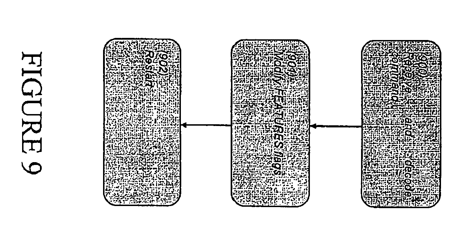

The operation of the CPAP device when connected to an upgrade key is shown in Figure 9. Upon receipt of the correct serial command from the upgrade key (900), the CPAP device sets an appropriate flag (901) in the 'FEATURE' flags, stored in non-volatile memory. These flags control what device features are enabled on the CPAP device. By setting one of the flags, the associated feature, such as humidifier operations, can be enabled. Once this is completed, the CPAP device restarts with the newly enabled features (902).

### 5.3.4 Process Flow

The upgrade from no humidity to cold passover may require a mechanical key to remove the shroud.

To upgrade from cold to heat an authentic electronic key may be inserted into the serial port.

If the electronic key is present when the unit is turned on, the unit will activate the heater and then the key will be deactivated. Once the electronic key is deactivated, it cannot be used again in another unit.

The electronic key can then be removed and the heater will remain active.

The electronic key may be a generic key or a unique internal and external access key.

Security may be also maintained by the circuitry in the key being too complicated to reproduce.

A database is not needed in the example where the key isn't matched uniquely to the unit.

### 6. Removable Heater Plate

The CPAP device is preferably supplied with a plastic cradle where a heater plate currently is that is below the water chamber 106 in Figure 3. The plastic cradle has all of the mounting and springing arrangement that the current heater plate has. The cradle also has an electrical connector and clips. A second part consisting of the pressed metal heater plate top surface with the element, thermistor and thermal cutout bonded to it is then supplied in the upgrade kit. The dealer can then remove the shroud and plug the heater plate into the cradle. The plastic cradle means that the fixings and spring mechanism can be preassembled inside the unit so that the heater plate can be installed without tools and without the need to disassemble the unit.

It will be appreciated that while in the preferred embodiment the unhumidified CPAP configuration utilises a shroud over the heater plate, this is not required. The heater plate could be rendered in operable by any of the methods described above, but with a water chamber or humidifier (see 106 Figure 3) in place in both configurations.

### 7. Cold Passover

The system in Figure 3 includes a water chamber 106 that may be operated in both humidified and non humidified modes. For example the heater plate need not be initially supplied or the heater plate may be supplied but not activated.

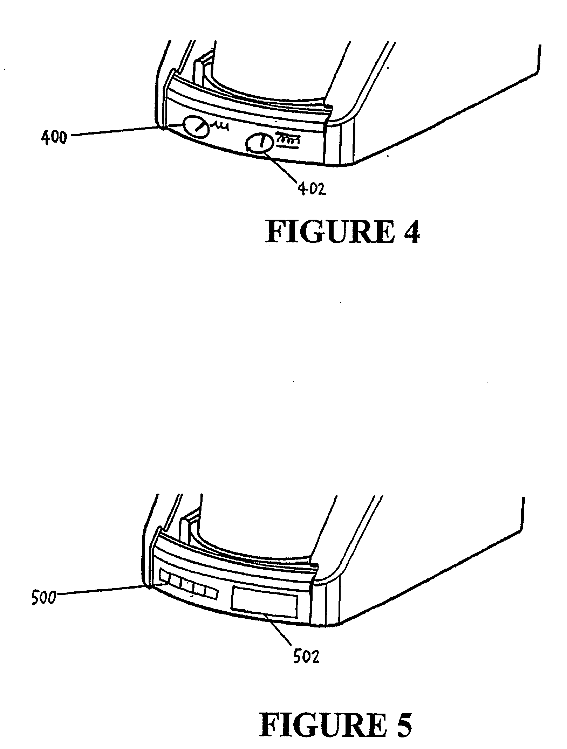

Activating the heater plate could occur for example, when;
1. the heater plate is controlled by a rotary potentiometer 108. The potentiometer 108 may be incapacitated by a lock to physically prevent the potentiometer from turning. Upgrade to active humidification requires a second key to remove the potentiometer lock and activate the heater.
2. the heater plate is controlled by a setting in a menu accessed by buttons on the CPAP device. Upgrade to active humidification in this case might require a PIN number to activate the heater plate. Referring to Figure 5 such a key pad 500 is shown.

### 8. Heated Tube

The CPAP device and accompanying system could include a heated gases delivery tube to deliver gases to a patient and minimise condensation of a humidified gases in the delivery tube. Again, this might be incapacitated initially.

The heated tube control may be activated by a rotary potentiometer, which is locked to physically prevent the potentiometer from turning. Upgrade requires a third key to remove the heated tube potentiometer lock and activate the heated tube.

Alternatively, the heated tube control may be activated by settings in a menu accessed by buttons such as those on the front of the CPAP device. Again, upgrade would require a PIN number to activate the heated tube.

Example upgrade kits might:
1. Cold passover kit: Contains a chamber, key #1 to remove cover.
2. Heated humidification kit: Contains chamber, key #1 to remove cover and key #2 to remove the potentiometer lock OR a PIN number to activate the heater.
3. Heated tube kit: Contains chamber, key #1 to remove cover, key #2 to remove the potentiometer lock OR a PIN number to activate the heater, and key #3 to remove the heated tube potentiometer lock OR a second PIN number.

### 9. Other Functionality Upgrades

The electronic or mechanical key may also be used to upgrade other functionality. For example, it might be desirable to store and/or display data in relation to patient compliance, or use of the treatment CPAP device by the patient.

Referring to Figure 5 we see a screen 502 for display of such compliance data. Depending on the key used example levels of functionality are given below:
Option 1: Machine displays no information.
Option 2: Machine displays machine run time only.
Option 3: Machine displays summary data on the display. For example, average hours complied per night used.
Option 4: Full compliance data download. All the day to data stored in the machine can be downloaded to a PC for analysis. Summary data would also be displayed.

It would be possible to activate any of these options by entering a suitable PIN number, or any other method, for example previously described.

This method could also apply to upgrading any function of the machine for example different levels of delivered pressure for inhalation versus exhalation and automatically calibrating the pressure level depending on symptoms exhibited by a patient.

## Claims

1. A breathing assistance apparatus comprising:
a gas delivery device adapted to provide gas at a substantially positive mean pressure;
a communication port adapted to communicate with an external device;
wherein said device is configurable to at least two configurations,
a first configuration where said gas delivery device has a predetermined functionality in a first state, and
a second configuration where said gas delivery device has a predetermined functionality in a second state, said second configuration being activated by said external device.

2. A breathing assistance apparatus according to claim 1 wherein said external device is rendered inoperable if said second configuration is activated.

3. A breathing assistance according to claim 1 or 2 wherein the gas delivery device includes a humidifier for humidifying said gas.

4. A breathing assistance apparatus according to any one of claims 1 to 3 wherein said predetermined functionality is active humidification of said gas.

5. A breathing assistance apparatus according to any one of claims 1 to 4 wherein a portion of said apparatus is excluded from use in said first configuration, whereby the integration of use of said portion to said apparatus corresponds to said second configuration.

6. A breathing assistance apparatus according to claim 4 wherein said second configuration is enabled by connecting a software or hardware upgrade key to said apparatus.

7. A breathing assistance apparatus according to claim 6 wherein said upgrade key includes an electronic circuit or controller configured to provide a predetermined response data stream to a received predetermined request data stream.

8. A breathing assistance apparatus according to claim 7 wherein said electronic circuit or controller includes at least one register indicating whether said electronic circuit or controller has been used and if it so said circuit or controller is rendered inoperative for subsequently activating said second configuration.

9. A breathing assistance apparatus according to claim 8 wherein said register includes the serial number of the apparatus said electronic circuit or controller was used with.

10. A breathing assistance apparatus according to any one of claims 1 to 9 wherein said apparatus further comprises a conduit between said gas delivery device and a patient and said predetermined functionality is active humidification and the provision of a heater within or incorporated with said conduit.

11. A breathing assistance apparatus according to claim 9 wherein said predetermined functionality relates to the heating said gas and said second configuration relates to energising said heater to heat said conduit and/or said gas directly.

12. A breathing assistance apparatus according to any one of claims 1 to 3 wherein said predetermined functionality relates to storage or display of data relating to the use of said apparatus and said second configuration relates to energising a display to indicate said use.

13. A breathing assistance apparatus according to any one of claims 1 to 3 wherein said predetermined functionality relates to the pressure level of said gases delivered to the patient and said second configuration relates to providing either a continuous or stepwise correction in said pressure level.

14. A method of delivering respiratory gases to a patient comprising the steps of:
providing gases at a substantially positive mean pressure from a gases supply device;
said gases supply device communicating with an external device to enable the switching of said gases supply device between at least two configurations, a first configuration with a predetermined functionality in a first state, and
a second configuration with said predetermined functionality in a second state capable of being enabled by said external device.

15. A method of delivering respiratory gases to a patient as claimed in claim 14 wherein said method includes the step of said external device being rendering inoperative if said second configuration is enabled.
